# EUROPEAN PATENT APPLICATION

(11) **EP 1 911 872 A2**
(43) Date of publication of application: **16.04.2008**
(21) Application number: 07004899.6
(22) Date of filing: 09.03.2007
(51) Int. Cl.: D06F 58/20

(54) **Scent supply apparatus and washing/drying machine having the same**

(30) Priority: 25.09.2006 KR 20060093181
(71) Applicant: LG Electronics Inc., Seoul, 150-721 (KR)
(72) Inventor: Kim, Kyeong-Hwan, Gangseo-Gu Seoul (KR); Jeon, Si-Moon, Seocho-Gu Seoul (KR); Woo, Kyung-Chul, Yangcheon-Gu Seoul (KR)
(74) Representative: Vossius & Partner

(57) **Abstract**

Provided are a scent supply apparatus (100) and a washing/drying machine including the same. The scent supply apparatus of a washing/drying machine including a tub (21) in a casing (11), and a guide duct (48) carrying air into the tub, is detachably installed at one side of the guide duct and supplies a scent into the tub. Because the scent supply apparatus is installed in the guide duct, the scent may be forcibly supplied into the washing/drying machine by the air being introduced into the tub from the guide duct. Accordingly, the scent may be actively supplied into the washing/drying machine, without depending only on its own diffusion, thereby improving scent supply efficiency. Also, when the scent source is exhausted and there is a need for replenishment thereof, a user may easily perform the replenishment of a scent source.

## Description

The present invention relates to a washing/drying machine, and more particularly, to a washing/drying apparatus having a scent supply apparatus detachably mounted to a guide duct carrying the air into the washing/drying apparatus and continuously supplying a scent if necessary.

One example of a scent supply apparatus includes a scent supply structure of a washing machine disclosed in utility model application, Korean Utility No. 20-0165264. In the scent supply structure, a holder is integrally formed at a lower surface of a door to be placed above a washing tub, the holder includes a receiving space and a plurality of through holes communicating with the receiving space, and the receiving space of the holder contains a membrane scent diffusing the scent.

Also, another example of the scent supply apparatus includes a fragrance supply apparatus of a washing machine disclosed in Korean Patent No. 10-0377411. The fragrance supply apparatus is installed at a predetermined location of the washing machine to supply a fragrance, and a heating unit installed near the fragrance supported by the holder to facilitate scent supply of the fragrance. Besides, a scent supply apparatus supplying a scent to a washing machine or a clothes processing apparatus is disclosed in many documents.

However, the conventional scent supply apparatus does not forcibly supply a scent (e.g., forcible supply of a scent by the air flowing through a guide duct), but supplies the scent only based on its own diffusion, which lowers scent supply efficiency.

Also, the conventional scent supply apparatus causes inconvenience because it is not easy for a user to re-supply a scent source when the scent source is exhausted and needs to be re-supplied. That is, in the case of Korean Utility No. 20-0165264, the user has to open the door and insert a membrane scent to the holder. Also, in the case of Korean Patent No. 10-0377411, the user has to unscrew a screw and take out the fragrance supply apparatus installed at a top cover.

Accordingly, the present invention is directed to a scent supply apparatus and a washing/drying machine having the same that substantially obviates one or more of the problems due to limitations and disadvantages of the related art.

An advantage of the present invention is to provide a scent supply apparatus having high scent supply efficiency.

Another advantage of the present invention is to provide a scent supply apparatus in which a scent source may be supplemented.

Another advantage of the present invention is to provide a washing/drying machine having a scent supply device having the above-described features.

Additional features and advantages of the invention will be set forth in the description which follows, and in part will be apparent from the description, or may be learned by practice of the invention. The objectives and other advantages of the invention will be realized and attained by the structure particularly pointed out in the written description and claims hereof as well as the appended drawings.

To achieve these and other advantages and in accordance with the purpose of the present invention, as embodied and broadly described herein, there is provided a scent supply apparatus of a washing/drying machine including a tub in a casing and a guide duct carrying the air into the tub. The scent supply apparatus includes a means for detachably installing the scent supply apparatus at one side of the guide duct and a means for supplying a scent into the tub.

In another aspect of the present invention, there is also provided a washing/drying machine including: a casing; a tub; a drum; a drive motor driving the drum; a guide duct guiding the air into the tub; and a scent supply apparatus detachably mounted to one side of the guide duct and supplying a scent into the tub.

It is to be understood that both the foregoing general description and the following detailed description are exemplary and explanatory and are intended to provide further explanation of the invention as claimed.

The accompanying drawings, which are included to provide a further understanding of the invention and are incorporated in and constitute a part of this specification, illustrate embodiments of the invention and together with the description serve to explain the principles of the invention.

In the drawings:
FIG. 1 is a cross-sectional view of a washing/drying machine including a scent supply apparatus installed at one side of a guide duct according to one embodiment of the present invention;
FIG. 2 is a view partially illustrating a front side of the washing/drying machine including the scent supply apparatus of FIG. 1;
FIG. 3 is a cross-sectional view of the scent supply apparatus installed at the guide duct of FIG. 1;
FIG. 4 is a view illustrating a scent supply apparatus according to a first modified embodiment;
FIG. 5 is a view illustrating a scent supply apparatus installed at a guide duct according to a second modified embodiment;
FIG. 6 is an exploded perspective view of the scent supply apparatus of FIG. 5;
FIG. 7 is a view taken along line VII-VII of FIG. 5;
FIG. 8 is a perspective view of a horizontal wall of FIG. 7;
FIG. 9 is a perspective view of a piston of FIG. 7;
FIG. 10 is a view illustrating a scent supply apparatus installed at a guide duct according to a third modified embodiment of the present invention;
FIG. 11 is an enlarged view of a cartridge holder of FIG. 10;
FIG. 12 is a perspective view of a cartridge of FIG. 10; and
FIG. 13 is a view taken along line XIII-XIII of FIG. 10.

Reference will now be made in detail to the embodiments of the present invention, examples of which are illustrated in the accompanying drawings.

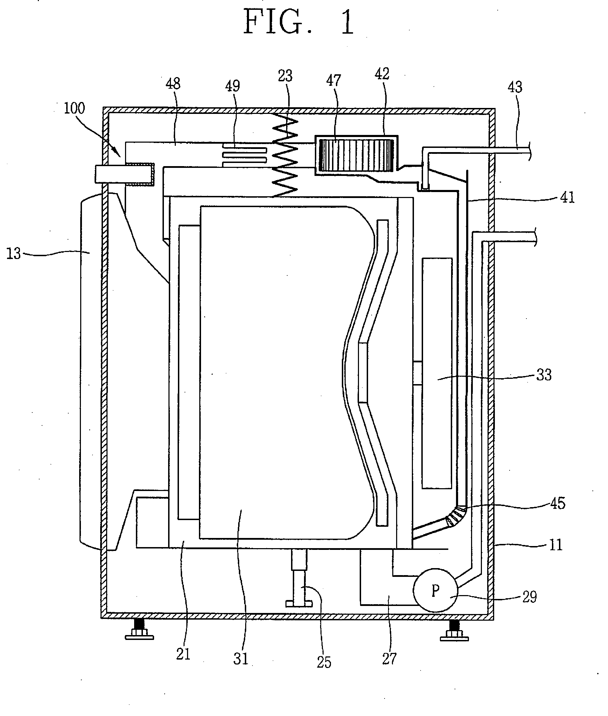

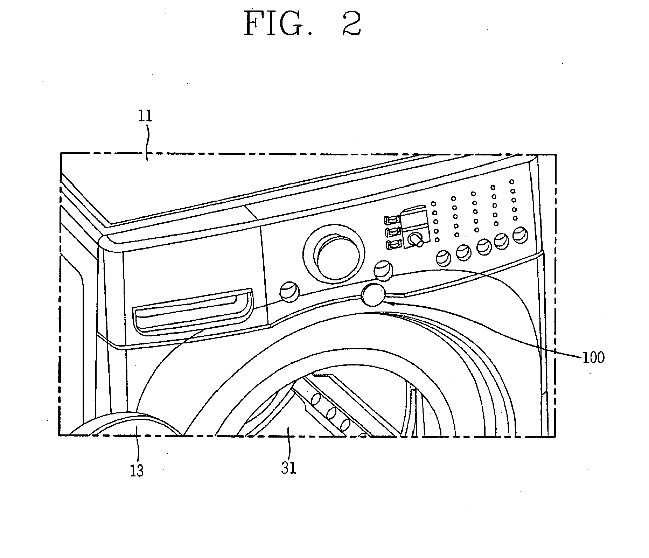

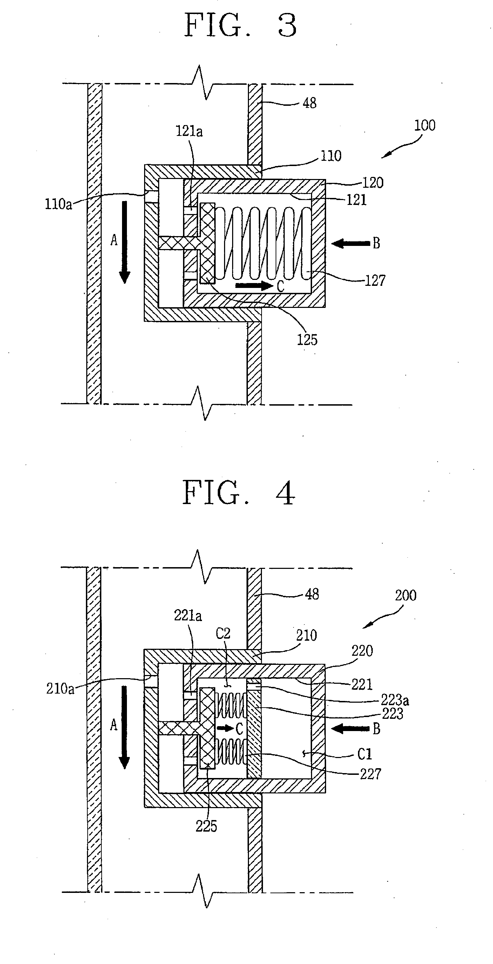

FIG. 1 is a cross-sectional view of a washing/drying machine including a scent supply apparatus installed at one side of a guide duct according to one embodiment of the present invention, FIG. 2 is a view partially illustrating a front side of the washing/drying machine including the scent supply apparatus of FIG. 1, and FIG. 3 is a cross-sectional view of the scent supply apparatus installed at the guide duct of FIG. 1.

Referring to FIG. 1, a washing/drying machine including a scent supply apparatus includes a casing 11, a tub 21 disposed inside the casing 11 and receiving washing water therein, a drum 31 disposed in the tub 21 to be rotatable about a rotary shaft disposed along an almost horizontal direction, a drive motor 33 driving the drum 31, a guide duct 48 carrying the air into the tub 21, and a scent supply apparatus 100 detachably installed at one side of the guide duct 48 and supplying a scent into the tub 21.

The casing 11 forms an exterior of the washing/drying machine, and has an entrance for input and output of laundry at a front side thereof, and a door 13 provided at one side of the entrance to open/close the entrance.

The tub 21 has a cylindrical shape having one side that is open and that is oriented such that its open side corresponds to the entrance. A spring member 23 and a damper 25 are installed under or on the tub 21, respectively so as to buffer and support the tub 21. A drain pipe 27 and a drain pump 29 are provided at one lower portion of the tub 21 so as to drain washing water.

A condensing pipe 41 is installed at a rear region of the tub 21 such that one end of the condensing pipe 41 communicates with the tub 21, and the other end thereof upwardly extends. A blower fan 47 is provided at an upper end of the condensing pipe 41 to draw out the air within the tub 21 through the condensing pipe 41.

A discharge side of the blower fan 47 is coupled to the other end of a guide duct 48 having one end communicating with an upper front side of the tub 21, and the guide duct 48 includes therein a heater 49 so as to heat the air.

The condensing pipe 41 has an "L" shape so that water in the air drawn out from the tub 21 condenses and can flow therein and has a fan coupling part 42 at an upper region for the coupling with the blower fan 47. A lower end of the condensing pipe 41 is connected to the other end of a connection corrugated pipe 45 having one end communicating with the tub 21.

By such a structure, when the blower fan 47 is driven as spin drying is terminated and drying is initiated, the air is taken out from the tub 21 and flows upwardly along the condensing pipe 41. The air flowing upwardly flows along the guide duct 48 and, is heated by the heater, and then is introduced into the tube 21.

The hot air guided into the tub 21 absorbs moisture of the laundry and flows along the condensing pipe 41. The air is cooled and condensed by condensing water supplied through a condensing water supply pipe 43. The dry cool air from which moisture has been removed is heated by the heater 49, and the heated dry air is guided into the tub 21 to absorb moisture from the laundry and then is condensed in the condensing pipe 41, thereby performing a drying operation of the laundry.

Referring to FIGS. 2 and 3, the scent supply apparatus 100 is detachably installed at the guide duct 48 such that it passes through a front side of the casing 11 above the door 13 from the outside across a direction in which the air flows within the guide duct 48. Here, the arrow A of FIG. 3 denotes a direction of the air flowing within the guide duct 48.

Here, the scent supply apparatus 100 may be detachably installed at the guide duct 48 such that it passes through a top side of the casing 11 from the outside of the guide duct 49 along the flowing direction of the air flowing within the guide duct 48.

Because the scent supply apparatus 100 may be detached/attached from/to the guide duct 48 from the outside, a user may easily detach/attach the scent supply apparatus 100 from/to the guide duct 48.

The scent supply apparatus 100 includes a cartridge holder 110 installed at the guide duct 48, and a cartridge 120 detachably coupled to the cartridge holder 110 and receiving a scent source.

The cartridge holder 110 includes a through hole 110a through which the air may be diffused to the guide duct 48. The cartridge holder 110 prevents the air flowing within the guide duct 48 from being leaked to the outside of the washing/drying machine even when the cartridge 120 is absent.

The cartridge 120 includes a receiving chamber 121 receiving a scent source, a discharge hole 121 a installed at one side of the receiving chamber 121 for discharging the scent, and an opening/closing unit opening/closing the discharge hole 121a. Here, the scent source has an aroma scent or a membrane scent. Of course, a scent source having a scent according to a user's taste may be used in addition to the above-mentioned scents.

The opening/closing unit includes a piston 125 having a portion protruding to the outside of the cartridge 120 to contact the cartridge holder 110, and opening/closing the discharge hole 121a, and an elastic member 127 elastically biasing the piston 125.

An operation of the scent supply apparatus will now be described with reference to FIG. 3.

When a user presses the cartridge 120 in a direction of arrow B, the elastic member 127 is compressed in a direction of arrow C, opening the discharge hole 121a that was closed by the piston 125. Here, a scent of the scent source received in the receiving chamber 121 is discharged through the discharge hole 121 a. The discharge air flows to the guide duct 48 through the through hole 110a of the cartridge holder 110, and then is supplied into the washing/drying machine along the air being supplied to the tub through the guide duct 48.

If the user does not press the cartridge 120, the piston 124 blocks the discharge hole 121a by a restoration force of the elastic member 126, so that the scent is not supplied any longer.

Because the scent supply apparatus 100 is installed in the guide duct 48, the scent may be forcibly supplied into the washing/drying machine by the air being introduced into the tub 21 from the guide duct 48. Accordingly, the scent may be actively supplied into the washing/drying machine, without depending only on diffusion, thereby improving scent supply efficiency.

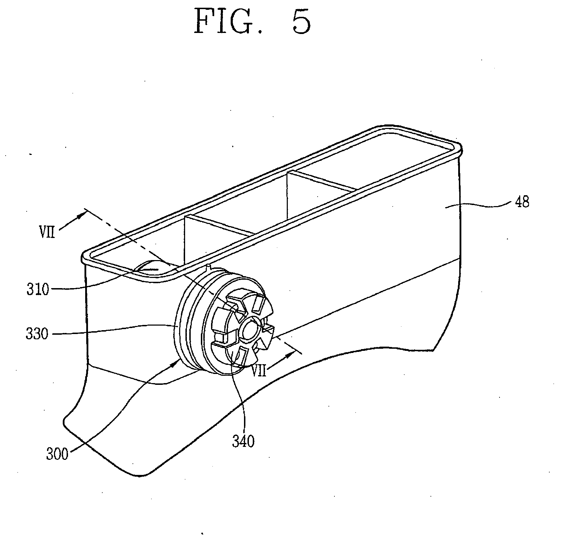

FIG. 4 is a view illustrating a scent supply apparatus according to a first modified embodiment of the present invention. Here, the arrow A denotes a direction in which the air flows within the guide duct.

The scent supply apparatus 200 according to the first modified embodiment includes a cartridge holder 210 installed at the guide duct 48, and a cartridge 220 detachably coupled to the cartridge holder 210 and receiving a scent source.

The cartridge 220 includes a receiving chamber 221, a discharge hole 221 a formed at one side of the receiving chamber 221 for discharging a scent, and an opening/closing unit opening/closing the discharge hole 221a. A through hole 210a through which a scent is discharged to the guide duct 48 is formed at the cartridge holder 210.

The opening/closing unit includes a horizontal wall 223 installed within the cartridge 220 and dividing the receiving chamber 221 into a first chamber C1 and a second chamber C2, a piston 225 having a portion protruding to the outside of the cartridge 220 to contact the cartridge holder 210, and opening/closing the discharge hole 221a, and an elastic member 227 elastically biasing the piston 225. Here, the first chamber C1 receives a scent source, and the horizontal wall 223 includes a scent hole 223a.

An operation of the scent supply apparatus according to the first modified embodiment will now be described with reference to FIG. 4.

When a user presses the cartridge in a direction of arrow B, the elastic member 227 is compressed in a direction of arrow C, opening the discharge hole 221a that was closed by the piston 225. Here, a scent of the scent source received in the first chamber C1 flows to the second chamber C2 through the scent hole 223a, and the scent is discharged through the discharge hole 221a. The discharge scent flows to the guide duct 48 through the through hole 210a and then is supplied into the washing/drying machine along with the air supplied to the tub 21 through the guide duct 48.

When the user does not press the cartridge 220, the piston 225 blocks the discharge hole 221 by a restoration force of the elastic member 227, so that the scent is not supplied any longer,

Because the scent supply apparatus 200 is installed within the guide duct 48, in addition to its own diffusion, the scent may be forcibly supplied into the washing/drying machine by the air being introduced into the tub 21 from the guide duct 48. Thus, the scent may be actively supplied into the washing/drying machine, thereby improving the scent supply efficiency.

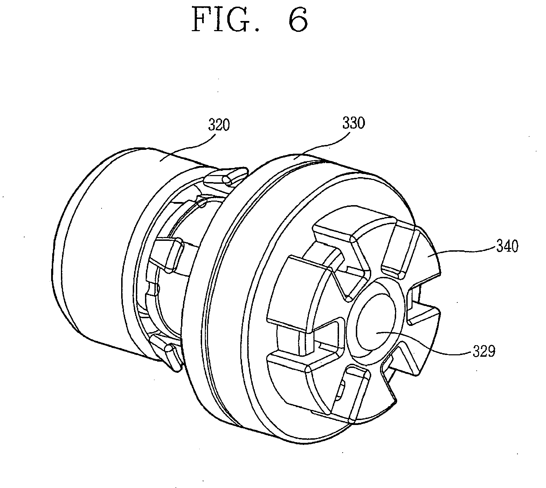

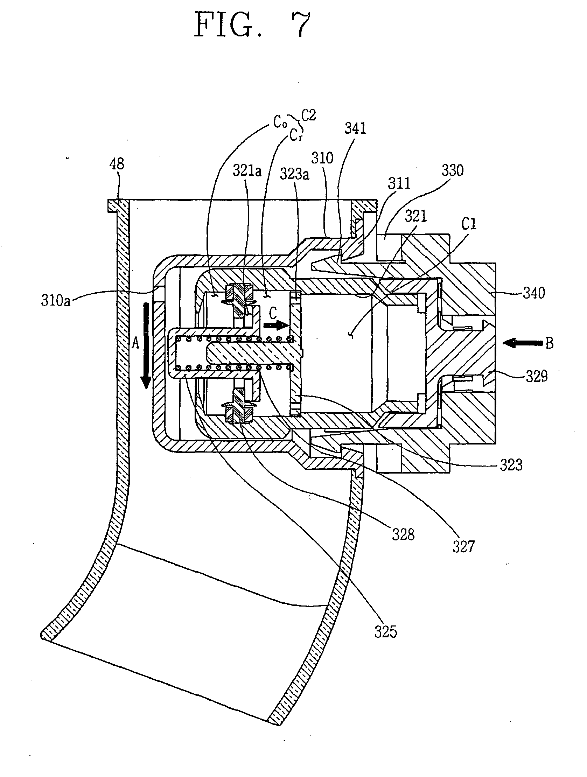

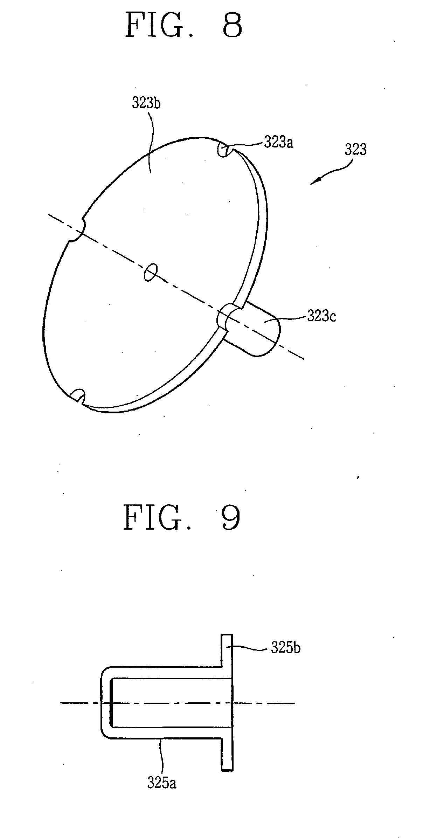

FIG. 5 is a view illustrating a scent supply apparatus installed at a guide duct according to a second modified embodiment, FIG. 6 is an exploded perspective view of the scent supply apparatus of FIG. 5, FIG. 7 is a view taken along line VII-VII of FIG. 5, FIG. 8 is a perspective view of a horizontal wall of FIG. 7, and FIG. 9 is a perspective view of a piston of FIG. 7. Here, the arrow A denotes a direction in which the air flows within the guide duct.

Referring to FIGS. 5 through 7, a scent supply apparatus 300 according to the second modified embodiment includes a cartridge holder 310 installed at the guide duct 48, a cartridge 320 detachably mounted to the cartridge holder 310 and receiving a scent source, a cartridge sealer 330 installed between the cartridge holder 310 and the cartridge 320, and an outer button 340 that detaches or attaches the cartridge 320 from or to the cartridge holder 310.

The cartridge holder 310 includes a through hole 310a through which the scent is discharged into the guide duct 48.

The cartridge 320 includes a receiving chamber 321, a discharge hole 321 a formed at one side of the receiving chamber 321 for discharging a scent, and an opening/closing unit opening/closing the discharge hole 321 a.

The opening/closing unit includes a horizontal wall 323 installed in the cartridge 320 and dividing the receiving chamber 321 into a first chamber C1 and a second chamber C2, a piston 325 having one portion protruding to the outside of the cartridge 320 to contact the cartridge holder 310 and opening/closing the discharge hole 321 a, an elastic member 327 elastically biasing the piston 325, a sealing member 328 opening/closing the discharge hole 321 a formed between the cartridge 320 and the piston 325, and an inner button 329 moving the piston 325.

Referring to FIGS. 7 and 8, the horizontal wall 323 includes a plate 323b dividing the inside of the cartridge 320 into the first chamber C1 and the second chamber C2, a guide protrusion 323c protruding from a central portion of the plate 323b to a predetermined length, and a scent hole 323a penetratingly formed in an outer circumferential surface of the plate 323b.

The first chamber C1 receives a scent source that moves to the second chamber C2 through the scent hole 323.

Referring to FIGS. 7 and 9, the piston 325 includes a contact portion 325a contacting a bottom of the cartridge holder 310, a partition wall portion 325b formed around an outer circumferential surface of the contact portion 325a and dividing the second chamber C2 into a replenishment chamber Cr and an outside chamber Co. A sponge (not shown) is installed in the replenishment chamber Cr to control the amount and rate of the scent being discharged, so that the scent may be evenly distributed.

The elastic member 327 is a spring installed in the contact portion 325a such that the guide protrusion 323c passes through the spring. When a force applied to the cartridge 320 by the elastic member 327 is removed, the cartridge 320 returns to its initial location.

The inner button 329 is installed outside the cartridge 320 and is a button pressed by the user to allow the cartridge 320 to make relative motion with respect to the cartridge holder 310, and thus make the guide protrusion 323c slide inside the contact portion 325a.

The sealing member 328 is installed at an inner surface of the cartridge 320 and forms the discharge hole 321 a between the sealing member 328 and the partition wall portion 325b when the guide protrusion 323c slides inside the contact portion 325a. The replenishment chamber Cr and the outside chamber Co selectively communicate with each other through the discharge hole 321 a.

The cartridge sealer 330 closely attaches the outer button 340 to an outer circumferential surface of the cartridge holder 310 and provides sealing to prevent the scent from being diffused to the outside.

The outer button 340 is installed to surround an outer circumferential surface of the inner button 328 and attaches or detaches the cartridge 320 to or from the cartridge holder 310.

An operation of the scent supply apparatus according to the second modified embodiment will now be described with reference to FIG. 7.

When a user presses the inner button 329 in a direction of arrow B, the elastic member 327 is compressed in a direction of arrow C, and the cartridge 320, the sealing member 328 and the horizontal wall 323 move integrally until the cartridge 320 is stopped by being blocked by the bottom of the cartridge holder 310.

Here, the contact portion 325a of the piston 325 does not move because it is blocked by the bottom of the cartridge holder 310, the sealing member 328 is separated from the horizontal wall 323, thereby forming the discharge hole 321 a. A scent of the scent source received in the replenishment chamber Cr is discharged to the guide duct 48 via the outside chamber Co and the through hole 310a. The scent discharged to the guide duct 48 is supplied into the washing/drying apparatus with the air being supplied to the tub 21 through the guide duct 48.

When the user does not press the inner button 329 any more, the cartridge 320 is moved to an initial location by a restoration force of the elastic member 327 and thus the discharge hole 321 a disappears, so that the scent is no longer supplied. Of course, if the continuous supply of the scent is needed, a hole allowing communication between the replenishment chamber Cr and the cartridge holder 310 is formed, so that the scent of the scent source received in the replenishment chamber Cr may be continuously supplied to the guide duct 48 through the through hole 310a.

When the inner button 329 is pressed and then released, the replenishment chamber Cr becomes empty as the scent of the scent source of the replenishment chamber Cr is discharged, but then the replenishment chamber Cr is continuously replenished with the scent source from the first chamber C1 through the scent hole 323a of the horizontal wall 323.

When there is a need for replenishing the first chamber C1 with the scent source as the scent source within the first chamber C1 has been exhausted, the user presses and turns the outer button 340 first. Here, a hook 341 of the outer button 340 is separated from a stopping projection 311 of the cartridge holder 310, and thus the scent supply apparatus 300 is separated from the cartridge holder 310.

Next, when the outer button 340 of the separated scent supply apparatus 300 is rotated in an unscrewing direction of a screw, the inner button 329 is rotated together, and thus the inner button 329 is separated from the cartridge 320. In this case, the first chamber C1 is opened, so that the first chamber C1 may be replenished with the scent source.

Then, when the outer button 340 is rotated in a direction of fastening the screw on the cartridge 320 after the replenishment of the scent source, the inner button 329 is rotated together, and is coupled to the cartridge 320.

Thereafter, when the outer button 340 is pressed, inserted in the cartridge holder 310, the hook 341 of the outer button 340 is caught by the stopping projection 311 of the cartridge holder 310.

Here, the cartridge sealer 330 installed between the scent supply apparatus 300 and the cartridge holder 310 is compressed, thereby completing sealing between the scent supply apparatus 300 and the cartridge holder 310.

Because the scent supply apparatus 300 is installed in the guide duct 48, the scent may be forcibly supplied into the washing/drying machine by the air being introduced into the tub 21 from the guide duct 48. Thus, the scent may be actively supplied into the tub 21, without depending only on its own diffusion, thereby improving scent supply efficiency.

Also, when there is a need for replenishment of the scent source as the scent source is exhausted, the first chamber may be easily replenished with the scent source in the aforementioned manner.

Also, a user may select a desired time for the scent supply, so that the scent may be supplied only during the corresponding period of time. Accordingly, the consumption of the scent source is reduced, so that the replacement period is lengthened and the cost is reduced.

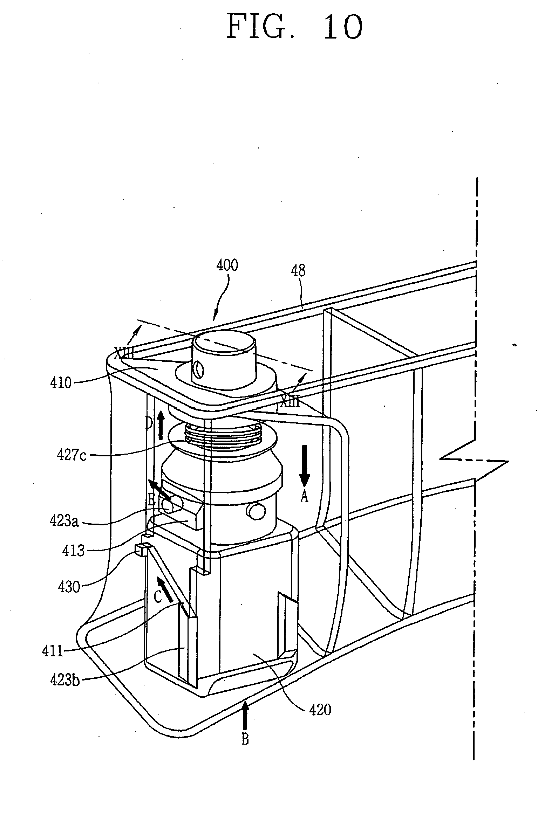

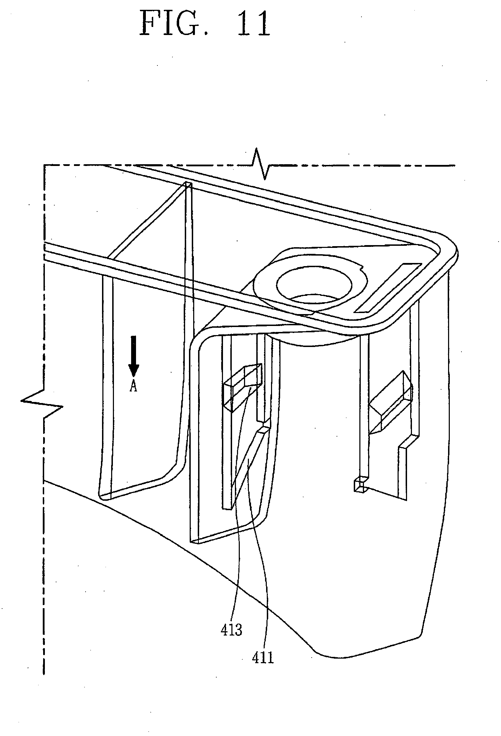

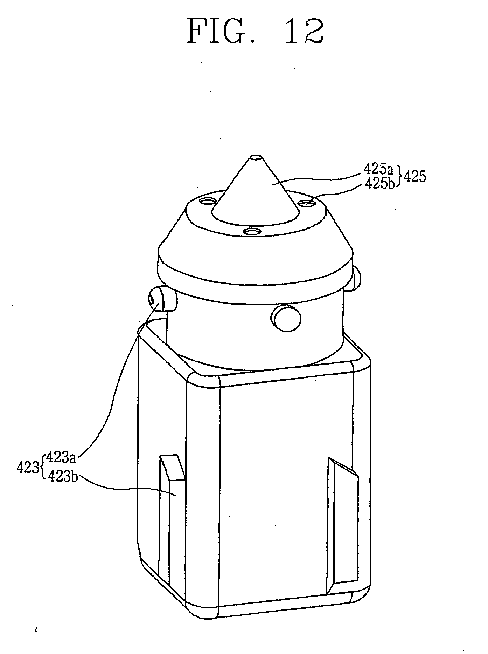

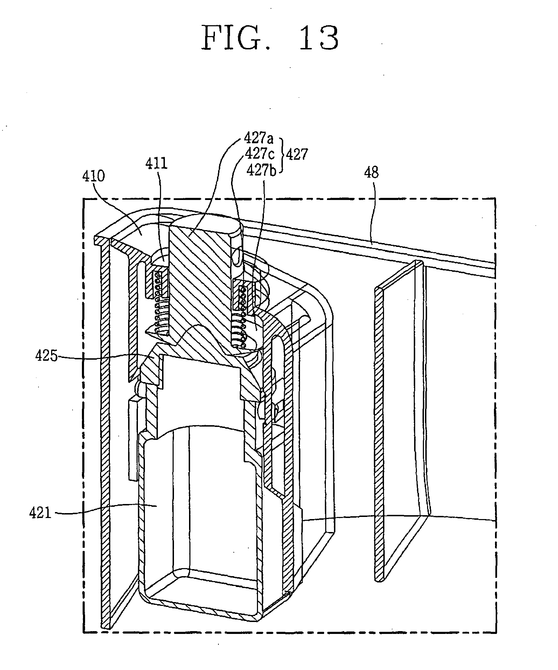

FIG. 10 is a view illustrating a scent supply apparatus installed at a guide duct according to a third modified embodiment of the present invention, FIG. 11 is an enlarged view of the cartridge holder of FIG. 10, FIG. 12 is a perspective view of the cartridge of FIG. 10, and FIG. 13 is a view taken along line XIII-XIII of FIG. 10. Here, the arrow A denotes a direction in which the air flows within the guide duct.

Referring to FIG. 10, a user opens the door 13 of Fig. 1 and put his hand into the guide duct 48 to detachably install a scent supply apparatus 400 according to the third modified embodiment of the present invention at the guide duct along a flow direction of the air. Of course, the scent supply apparatus 400 may be detachably installed at the guide duct 48 such that it is across the flowing direction of the air flowing in the guide duct 48.

The scent supply apparatus 400 includes a cartridge holder 410 installed at the guide duct 48, a cartridge 420 detachably coupled to the cartridge holder 410 and receiving a scent source, and a stopper 430 limiting a vertical movement of the cartridge 420 in the cartridge holder 410.

Referring to FIG. 11, the cartridge holder 410 is installed along the flow direction of the air flowing in the guide duct 48. An inclination rib 411 is formed at a side of the cartridge holder 410, and a slit 413 is formed above the inclination rib 411.

Such a cartridge holder 410 allows mounting of the cartridge 420 to be completed only by user's opening the door 130 and pushing up the cartridge 420 to the cartridge holder 410. The detailed operation will be described later.

Referring to FIGS. 12 and 13, the cartridge 420 includes a receiving chamber 421 receiving a scent source, a coupling portion 423 coupled to the cartridge holder 410, and a plug 425 having a discharge hole 425b through which a scent of a scent source received in the receiving chamber 421 is discharged.

The coupling portion 423 includes an inclination protrusion 423b sliding in mesh with the inclination rib 411 formed at the cartridge holder 410 and a coupling protrusion 423a coupled to or separated from the slit 413 formed at the guide duct 48 by the sliding of the inclination protrusion 423b.

Here, the scent supply apparatus 400 further includes an elastic portion 427 pushing the cartridge 420 out of the cartridge holder 410 when the coupling protrusion 423a of the coupling portion 423 is separated from the slit 413.

The elastic portion 427 includes a column-shaped coupling button 427a, a circular coupling plate 427b formed around the coupling button 427a, and a spring 427c inserted between the coupling plate 427b and the coupling plate 411 of the cartridge holder 410.

The plug 425 includes a cone-shaped protrusion 425a, and a discharge hole 425b penetrating formed around the cone-shaped protrusion 425a. The plug 425 is coupled to the elastic portion 427 as the cone-shaped protrusion 425a is inserted into a cone-shaped groove recessed at a lower side of the coupling button 427a. The scent discharged from the scent source received in the receiving chamber 421 is continuously provided to the guide duct 48 through the discharge hole 425b.

The stopper 430 is formed at an upper side of the inclination rib 411 formed on the cartridge holder 410, and limits a vertical sliding distance of the inclination protrusion 423b sliding in contact with the inclination rib 411.

A mounting method of the scent supply apparatus according to the third modified embodiment will now be described with reference to FIG. 10.

A user opens the door 13 of FIG. 1, puts his hand into the guide duct 48, and pushes up the cartridge 420 to the cartridge holder in a direction of arrow B.

In this case, the inclination protrusion 423b is slid on the inclination rib 411 in a direction of arrow C, and the coupling protrusion 423a is inserted in the slit 413 and simultaneously the spring 427c is compressed in a direction of arrow D. As the coupling protrusion 423a is inserted in the slit 413, the cartridge 420 is mounted to the cartridge holder 410, and the scent discharged through the discharge hole 425b of FIG. 12 of the mounted cartridge 420 is continuously supplied into the washing/drying machine through the guide duct 48.

When the cartridge 420 is pushed up further in the direction of arrow B in order to separate the cartridge 420 from the cartridge holder 410, the coupling protrusion 423a is separated from the slit 413 by being moved in a direction of arrow E. The spring 427c FIG. 13 compressed in a separated state pushes the cartridge 420 to a lower side of the cartridge holder 410, thereby separating the cartridge 420 from the cartridge holder 410.

When the plug 425 of FIG. 12 of the separated cartridge 420 is turned to release the screw, the plug 425 of FIG. 12 is separated from the cartridge 420, and the receiving chamber 421 of FIG. 13 is opened so that the user may replenish the receiving chamber 421 with a scent source.

Because the afore-described scent supply apparatus 400 is installed in the guide duct 48, the scent supply apparatus 400 may forcibly supply the scent into the washing/drying machine by the air being introduced to the tub from the guide duct 48. Accordingly the scent may be actively supplied to the tub 21 without depending only on diffusion, thereby improving the scent supply efficiency.

Also, when an existing scent source is exhausted and thus there is a need for replenishment of a scent source, the receiving chamber may easily replenished with the scent source in the afore-described method.

As described so far, because a scent supply apparatus according to embodiments of the present invention is installed in a guide duct, a scent may be forcibly supplied into a tub together along with the air passing the guide duct. Thus, the scent may be actively supplied into a washing/drying machine without depending only on diffusion, so that scent supply efficiency of the scent supply apparatus may be improved.

Also, when the scent source is exhausted and thus there is a need for replenishment thereof, the user may easily perform replenishment of a scent source.

Also, a user may set a time desired for scent supply, and thus the scent may be supplied only for the corresponding period of time, so that consumption of the scent source is reduced to lengthen the replacement period, and cost is reduced.

It will be apparent to those skilled in the art that various modifications and variation can be made in the present invention without departing from the spirit or scope of the invention. Thus, it is intended that the present invention cover the modifications and variations of this invention provided they come within the scope of the appended claims and their equivalents.

## Claims

1. A scent supply apparatus of a washing/drying machine including a tub in a casing and a guide duct carrying air into the tub, the scent supply apparatus comprising:
a means for detachably installing the scent supply apparatus at one side of the guide duct; and
a means for supplying a scent into the tub.

2. The scent supply apparatus of claim 1, wherein the scent supply apparatus is detachably installed from the outside or the inside of the guide duct, across the direction in which the air flows within the guide duct.

3. The scent supply apparatus of claim 1, wherein the scent supply apparatus is detachably installed from the outside or the inside of the guide duct along an air flow direction in which the air flows within the guide duct.

4. The scent supply apparatus of any one of the preceding claims, wherein the scent supply apparatus comprises:
a cartridge holder installed at the guide duct; and
a cartridge detachably coupled to the cartridge holder and receiving a scent source.

5. The scent supply apparatus of claim 4, further comprising a cartridge sealer installed between the cartridge holder and the cartridge.

6. The scent supply apparatus of claim 4 or 5, wherein the cartridge comprises:
a receiving chamber receiving a scent source;
a discharge hole formed at one side of the receiving chamber and discharging a scent; and
an opening/closing unit opening/closing the discharge hole.

7. The scent supply apparatus of claim 6, wherein the opening/closing unit comprises:
a piston having a portion protruding to the outside of the cartridge to contact the cartridge holder and to open/close the discharge hole; and
an elastic member elastically biasing the piston.

8. The scent supply apparatus of claim 6, wherein the opening/closing unit comprises:
a horizontal wall installed in the cartridge;
a piston having a portion protruding to the outside of the cartridge to contact the cartridge holder and to open/close the discharge hole; and
an elastic member elastically biasing the piston.

9. The scent supply apparatus of claim 7 or 8, wherein the opening/closing unit further comprises an inner button moving the piston.

10. The scent supply apparatus of claim 7 or 8, further comprising an outer button mounting or detaching the cartridge to or from the cartridge holder.

11. The scent supply apparatus of claim 8, 9 or 10, wherein the discharge hole is formed between the cartridge and the piston, and
the opening/closing unit further comprises a sealing member opening/closing the discharge hole.

12. The scent supply apparatus of any of claims 4 to 11, wherein the cartridge comprises:
a receiving chamber receiving a scent source;
a coupling portion coupled to the cartridge holder; and
a plug including a discharge hole through which a scent of the scent source received in the receiving chamber is discharged.

13. The scent supply apparatus of claim 12, wherein the coupling portion comprises:
an inclination protrusion sliding in contact with an inclination rib formed at the cartridge holder; and
a coupling protrusion coupled to or separated from a slit formed in the guide duct by the sliding of the inclination protrusion.

14. The scent supply apparatus of claim 13, further comprising an elastic portion pushing the cartridge out of the cartridge holder when the coupling protrusion is separated from the slit.

15. The scent supply apparatus of claim 13 or 14, further comprising a stopper installed at an upper side of the inclination rib.

16. A washing/drying machine comprising:
a casing;
a tub;
a drum;
a drive motor driving the drum;
a guide duct guiding air into the tub; and
a scent supply apparatus detachably mounted to one side of the guide duct and supplying a scent into the tub.
